# EUROPEAN PATENT SPECIFICATION

(11) **EP 2 285 413 B1**
(45) Date of publication and mention of the grant of the patent: **08.08.2012**
(21) Application number: 09766752.1
(22) Date of filing: 18.06.2009
(51) Int. Cl.: A61K 47/26, A61K 47/02, A61K 31/4704

(54) **A PHARMACEUTICAL COMPOSITION**
PHARMAZEUTISCHE ZUSAMMENSETZUNG
COMPOSITION PHARMACEUTIQUE

(30) Priority: 19.06.2008 JP 2008160084
(43) Date of publication of application: 23.02.2011
(73) Proprietor: Otsuka Pharmaceutical Co., Ltd., Tokyo 101-8535 (JP)
(72) Inventor: SUMIDA, Shun-ichiro, Osaka-shi Osaka 541-0045 (JP); ISHIKAWA, Shinichi, Osaka-shi Osaka 541-0045 (JP)
(74) Representative: von Kreisler Selting Werner
(86) International application number: PCT/JP2009/061553
(87) International publication number: WO 2009/154304

(56) References cited:
- WO-A1-2006/052018
- WO-A1-2008/050896

## Description

### TECHNICAL FIELD

The present invention relates to a pharmaceutical composition.

### BACKGROUND ART

Rebamipide [chemical name: (±)-2-(4-chlorobenzoylamino)-3-(2-quinolon-4-yl)propionic acid] is known as a useful antiulcer drug.

In addition, rebamipide has an increasing action of goblet cell density in eye, an increasing action of mucus in eye, and an increasing action of lacrimal fluid, and has been already known as an agent for treating dry eye, i.e. dry eye syndrome (WO 97/013515).

Rebamipide is soluble in an alkaline aqueous solution, but the solubility of rebamipide in a neutral solution is quite low. On the other hand, a high-pH eye drop is not suitable for a patient suffering from an injury in keratoconjunctiva such as dry eye. Additionally, even in case of an alkaline solution containing rebamipide, a crystal of rebamipide may be occasionally deposited and hence it is thought that the development of an aqueous ophthalmic product of rebamipide is difficult.

WO 97/013515 discloses a neutral aqueous suspension containing rebamipide. However, the suspension may form a precipitate layer when standing for a long period. Therefore, the suspension needs to be shaken well in order to be re-dispersed. In addition, such suspension product may be thought to have some demerits, for example suffering from blurred vision and making some white spots on the cloths when spilling the suspension, because the suspension product is a white ophthalmic suspension.

WO 2008/050896 discloses an aqueous suspension containing rebamipide wherein rebamipide is stably dispersed in a state of fine particles and additionally the fine particles are not re-agglutinated. The aqueous suspension containing rebamipide in WO 2008/050896 is improved about suspensibility compared with the production of the above-mentioned WO 97/013515. However, the aqueous suspension containing rebamipide in WO 2008/050896 is also necessary to be shaken well in order to be re-dispersed, since the suspension in WO 2008/050896 is a white ophthalmic suspension wherein rebamipide is not completely dissolved and it is impossible to avoid forming a precipitate layer of rebamipide when standing for a long period. Furthermore, the demerits such as suffering from blurred vision and making some white spots on the cloths when spilling the suspension, have not been solved in WO 2008/050896 yet.

WO 2006/052018 discloses an aqueous suspension containing crystalline rebamipide which comprises a mixture of at least one of the compounds selected from watersoluble polymers and surfactants, an aqueous acidic solution, and an aqueous solution containing a watersoluble salt of rebamipide, which is not necessary to be re-dispersed, has an enough transparency, and is a neutral suspension not to injure the keratoconjunctiva of a patient suffering from dry eye.

However, the aqueous suspension containing crystalline rebamipide in WO 2006/052018 has a problem of high production cost, since some expensive equipments such as a high-pressure homogenizer, a colloidmill, and a sonicator are required in the process, and the manufacturing process is troublesome, complicated, and long-term.

In general, to a conventional ophthalmic agent is added a preservative in order to prevent microbial contamination. For a patient suffering from corneal disorder such as dry eye, however, a preservative-free ophthalmic agent is required because a preservative is harmful to the patient. Vessels known for a preservative-free ophthalmic agent include a disposable plastic single-dose unit and a multi-use vessel equipped with a filter which can capture bacteria around the drip opening. However, the aqueous suspensions in the above-mentioned WO 2008/050896 and WO 2006/052018 are hard to pass through the filter, thus it is impossible to use the multi-use vessel, and it is necessary to use a comparatively-expensive single-dose unit.

Therefore, it has been desired to develop a pharmaceutical composition containing rebamipide, which is unnecessary to be re-dispersed, has an enough transparency, and exhibits neutral to weakly acidic pH not to injure the keratoconjunctiva of a patient suffering from dry eye, and a process for preparing such pharmaceutical composition at low cost without using expensive equipments.

### DISCLOSURE OF INVENTION

### (Problem to be solved by the invention)

An object of the present invention is to provide a pharmaceutical composition containing rebamipide, which is unnecessary to be re-dispersed, has an enough transparency, and exhibits neutral to weakly acidic pH not to injure the keratoconjunctiva of a patient suffering from dry eye. In detail, the pharmaceutical composition is an ophthalmic composition.

In addition, an object of the present invention is to provide a pharmaceutical composition which can be prepared at low cost without using expensive equipments.

### (Means to solve the problem)

The present inventors have extensively studied to reach the above object and then have found that a pharmaceutical composition comprising (1) rebamipide, (2) an amino sugar, and (3) an buffer agent, which has no cation, has a good re-dispersibility and an enhanced transparency. In addition, the present inventors have also found that the present pharmaceutical composition has an antimicrobial effectiveness. Based upon the new findings, the present invention has been completed.

The present invention provides pharmaceutical compositions and use thereof, as shown in the following [1] to [8].
[1] A pharmaceutical composition comprising (1) rebamipide, (2) an amino sugar, and (3) an buffer agent, which has no inorganic cation.
[2] The pharmaceutical composition of [1] wherein the amino sugar is at least one selected from the group consisting of D-glucosamine and meglumine.
[3] The pharmaceutical composition of [1] or [2] wherein the buffer agent is at least one selected from the group consisting of boric acid, phosphoric acid and an amino acid.
[4] The pharmaceutical composition of any one of [1] to [3], further comprising a pH adjuster.
[5] The pharmaceutical composition of any one of [1] to [4], further comprising a solubilizing agent.
[6] The pharmaceutical composition of [5] wherein the solubilizing agent is at least one selected from the group consisting of polyvinylpyrrolidone and macrogol.
[7] The pharmaceutical composition of any one of [1] to [6] wherein the pharmaceutical composition is an ophthalmic pharmaceutical composition.
[8] The pharmaceutical composition of any one of [1] to [7] for use in the treatment of dry eye by topical administration to the eye.

The pharmaceutical composition of the present invention is a composition comprising (1) rebamipide, (2) an amino sugar and (3) an buffer agent, which comprises no inorganic cation.

The pharmaceutical composition is preferably formulated as an aqueous liquid preparation, and more preferably used as an ophthalmic pharmaceutical composition.

The concentration of rebamipide in the pharmaceutical composition of the invention is about 0.1 % (W/V) to about 5 % (W/V), preferably about 0.5 % (W/V) to about 3 % (W/V), more preferably about 1 % (W/V) to about 2 % (W/V). For example, "1 % (W/V)" herein used means a concentration of 1 g per 100 mL.

The cation herein used refers to an inorganic cation, and includes, for example, a monovalent alkaline metal cation such as lithium ion, sodium ion, potassium ion, and cesium ion; and bivalent alkaline earth metal cation such as magnesium ion and calcium ion.
The term "a pharmaceutical composition has no inorganic cation" herein used means "a pharmaceutical composition does not virtually have an inorganic cation". In more detail, it means that the amount of an inorganic cation in the pharmaceutical composition is less than 15 mEq, preferably less than 1.5 mEq, more preferably less than 0.3 mEq.

The amino sugar herein used includes, for example, meglumine (i.e. N-methyl-D-glucamine), D-glucosamine, D-galactosamine, D-mannosamine, mycosamine, kanosamine, neosamine C, N-methyl-L-glucosamine, mycaminose, muramic acid, and streptamine.

Amongst these amino sugars, the preferable amino sugars are meglumine, D-glucosamine, D-galactosamine, D-mannosamine, mycosamine, kanosamine, neosamine C, N-methyl-L-glucosamine, mycaminose, muramic acid, and streptamine; especially meglumine and D-glucosamine are more preferable. The above amino sugars may be used as a single ingredient or a combination consisting of two or more ingredients.

The concentration of the amino sugar in the pharmaceutical composition of the invention is, for example, about 0.1 % (W/V) to about 15 % (W/V), preferably about 0.5 % (W/V) to about 10 % (W/V), more preferably about 1 % (W/V) to about 8 % (W/V).

The buffer agent herein used includes, for example, an acid which is not in cation form, such as boric acid, phosphoric acid, an amino acid, and an organic acid; preferably boric acid and phosphoric acid are used. The above buffer agents may be used as a single ingredient or a combination consisting of two or more ingredients.

The concentration of the buffer agent in the pharmaceutical composition of the invention is, for example, about 0.01 % (W/V) to about 4 % (W/V), preferably about 0.03 % (W/V) to about 3 % (W/V), more preferably about 0.05 % (W/V) to about 2 % (W/V).

The pharmaceutical composition of the invention may optionally contain a pH adjuster. The pH adjuster includes, for example, a conventional acid which is in cation-free form, such as hydrochloric acid, lactic acid, acetic acid, sulfuric acid, nitric acid, carbonic acid, phosphoric acid, and citric acid; preferably hydrochloric acid and citric acid are used. The pH adjusters may be used as a single ingredient or a combination consisting of two or more ingredients.

The pharmaceutical composition of the invention may optionally contain an isotonic agent in order to make the pharmaceutical composition isotonic to an aqueous tear. The isotonic agent is preferably a cation-free isotonic agent, which includes a conventional agent for ophthalmic solution such as mannitol, glycerin, polypropylene glycol, polyethylene glycol, maltose, sucrose, sorbitol, and glucose, more preferably a cation-free glycerin and sucrose are used. The isotonic agents may be used as a single ingredient or a combination consisting of two or more ingredients.

The pharmaceutical composition of the invention may optionally contain a solubilizing agent. The solubilizing agent includes, for example, a cation-free solubilizing agent. The cation-free solubilizing agent includes, for example, a polymer such as polyvinylpyrrolidone, macrogol (polyethylene glycol), polyvinyl alcohol, and hydroxypropylmethylcellulose; a surfactant such as polysorbate, polyoxyethylene hydrogenated castor oil, and polyoxyethylene-polyoxypropylene; a polyhydric alcohol such as polypropylene glycol; an organic acid such as benzoic acid and sorbic acid; an amino acid such as alginic acid, histidine, glycine, and lysine; and a xanthine derivative such as caffeine. The preferable solubilizing agent is polyvinylpyrrolidone, macrogol, polyvinyl alcohol, benzoic acid, sorbic acid, and alginic acid; especially, more preferable solubilizing agent is polyvinylpyrrolidone and macrogol. The solubilizing agents may be used as a single ingredient or a combination consisting of two or more ingredients.

The molecular weight of polyvinylpyrrolidone herein used is preferably not more than 200,000, more preferably not more than 40,000. The preferable polyvinylpyrrolidone includes, not limited thereto, polyvinylpyrrolidone (PVP, BASF, Grade: Kollidon^{®} 25): PVP (K-25), and polyvinylpyrrolidone (PVP, BASF, Grade: Kollidon^{®} 17PF): PVP (K-17PF). The concentration ratio of rebamipide and polyvinylpyrrolidone is preferably 20:1 - 1:20, more preferably 4:1 - 1:6.

The molecular weight of macrogol herein used is preferably not more than 50,000, more preferably not more than 10,000. The preferable macrogol includes, not limited thereto, macrogol 1500, macrogol 4000, macrogol 6000, and macrogol 20000. The concentration ratio of rebamipide and macrogol is preferably 20:1 - 1:20, more preferably 4:1 - 1:6.

In case of adding a solubilizing agent to the present composition, the concentration of solubilizing agent is in a range of generally about 0.01 (W/V) % - about 15 (W/V) %, preferably about 0.1 (W/V) % - about 10 (W/V) %, and more preferably about 0.5 (W/V) % - about 6 (W/V) %.

The pharmaceutical composition of the invention may optionally comprise a conventional preservative such as a quaternary ammonium salt (e.g. benzalkonium chloride, and benzethonium chloride, chlorhexidine gluconate, a paraoxybenzoate (e.g. methyl paraoxybenzoate and propyl paraoxybenzoate), and an alcohol compound (e.g. chlorobutanol and benzyl alcohol); and/or stabilizing agent such as an inorganic-cation-free ascorbic acid and tocopherol.

In case that the present pharmaceutical composition is an aqueous liquid preparation, the pH is about 3 - about 9, preferably about 7 - about 9, and the most preferably 7.7 - 9.

The ratio between (1) rebamipide and (2) an amino sugar comprised in the present composition depends on the type of ingredients used herein and the formulation type of the composition. The amount of (1) rebamipide is, but not limited to, for example, in about 0.01 - about 10 parts by weight, preferably about 0.05 - about 2 parts by weight, more preferably about 0.1 - about 1.5 parts by weight, and the most preferably 0.1 - 1 part by weight, per one part by weight of (2) an amino sugar.

A preferable pharmaceutical composition of the present invention is a pharmaceutical composition comprising (1) rebamipide, (2) meglumine and (3) boric acid, which comprises no inorganic cation. A more preferable pharmaceutical composition is a pharmaceutical composition comprising 0.5 % (W/V) to 3 % (W/V) rebamipide, 0.5 % (W/V) to 10 % (W/V) meglumine, and 0.03 % (W/V) to 3 % (W/V) boric acid, which comprises no inorganic cation. An even more preferable pharmaceutical composition is a pharmaceutical composition comprising 1 % (W/V) to 2 % (W/V) rebamipide, 1 % (W/V) to 8 % (W/V) meglumine, 0.05 % (W/V) to 2 % (W/V) boric acid, and a pH adjuster selected from the group consisting of citric acid and hydrochloric acid, which comprises no inorganic cation.

The most preferable pharmaceutical composition is the above-mentioned pharmaceutical composition further comprising polyvinylpyrrolidone as a solubilizing agent. The concentration of the solubilizing agent is preferably 0.5 (W/V) % to 6 (W/V).

### (Effect of the invention)

The transparency of the present pharmaceutical composition comprising (1) rebamipide, (2) an amino sugar, and (3) an buffer agent can be enhanced by not containing any inorganic cation. The present pharmaceutical composition is unnecessary to be re-dispersed, additionally it can be formulated into an aqueous preparation which has an enough transparency, and exhibits neutral to weakly acidic pH.

The present pharmaceutical composition has an antiseptic effect, thus it is expected to exhibit a preservative effect. Therefore, the present aqueous preparation is not necessary to comprise a conventional preservative (e.g. a quaternary ammonium salt such as benzalkonium chloride and benzethonium chloride; a cationic compound such as chlorhexidine gluconate; a paraoxy benzoate such as methyl paraoxybenzoate and propyl paraoxybenzoate; and an alcohol compound such as chlorobutanol and benzyl alcohol).

In addition, the present pharmaceutical composition is so stable, thus it is not necessary to add hereto any stabilizing agent (e.g. an inorganic-cation-free ascorbic acid and tocopherol).

The manufacturing process of the present pharmaceutical composition can be carried out at low cost, not via any troublesome process, without using any special dispersing/suspending device, which is an industrially great merit.

The present aqueous ophthalmic product can prevent an undesirable blurred vision of an applied patient, thus it is expected to markedly enhance the compliance of a patient suffering from dry eye, and it enables the use of a multi-use vessel equipped with an aseptic filter around the drip opening which is for a preservative-free ophthalmic agent, because it can be pass through an aseptic filter. Therefore, the present invention will provide a great amount of medicinal contribution.
In addition, the present pharmaceutical composition has an excellent transparency, thus there is no trouble of making some white spots on the cloths when spilling the composition.

### BEST MODE FOR CARRYING OUT THE INVENTION

The present invention is illustrated in more detail by the following examples, but should not be construed to be limited thereto.

### Example 1

| | |
|---|---|
| Rebamipide | 2 g |
| Polyvinylpyrrolidone (K-25) | 3 g |
| Boric acid | 1.5 g |
| Meglumine | 5.9 g |
| Glycerin | 0.722 g |
| Hydrochloric acid | q.s. (to pH=8.3) |
| Purified water | q.s. |
| Total | 100 mL |

| | |
|---|---|
| Note: q.s. means quantum sufficiat. | |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a light-yellowish to pale-yellowish transparent solution.

### Example 2

| | |
|---|---|
| Rebamipide | 2 g |
| Polyvinylpyrrolidone (K-25) | 3 g |
| Boric acid | 1 g |
| Meglumine | 4.3 g |
| Glycerin | 1.185 g |
| Hydrochloric acid | q.s. (to pH=8.3) |
| Purified water | q.s. |
| Total | 100 mL |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a light-yellowish to pale-yellowish transparent solution.

### Example 3

| | |
|---|---|
| Rebamipide | 2 g |
| Polyvinylpyrrolidone(K-25) | 1 g |
| Boric acid | 1.5 g |
| Meglumine | 6 g |
| Glycerin | 0.796 g |
| Hydrochloric acid | q.s. (to pH=8.5) |
| Purified water | q.s. |
| Total | 100 mL |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a colorless to light-yellowish transparent solution.

### Example 4

| | |
|---|---|
| Rebamipide | 2 g |
| Polyvinylpyrrolidone (K-25) | 6 g |
| Boric acid | 1.5 g |
| Meglumine | 5.9 g |
| Glycerin | 0.398 g |
| Hydrochloric acid | q.s. (to pH=8.3) |
| Purified water | q.s. |
| Total | 100 mL |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a light-yellowish to pale-yellowish transparent solution.

### Example 5

| | |
|---|---|
| Rebamipide | 1 g |
| Polyvinylpyrrolidone (K-25) | 3 g |
| Boric acid | 1.5 g |
| Meglumine | 4.7 g |
| Glycerin | 0.991 g |
| Hydrochloric acid | q.s. (to pH=8.0) |
| Purified water | q.s. |
| Total | 100 mL |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a light-yellowish to pale-yellowish transparent solution.

### Example 6

| | |
|---|---|
| Rebamipide | 1 g |
| Polyvinylpyrrolidone (K-25) | 3 g |
| Boric acid | 1 g |
| Meglumine | 3.4 g |
| Glycerin | 1.389 g |
| Hydrochloric acid | q.s. (to pH=8.0) |
| Purified water | q.s. |
| Total | 100 mL |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a light-yellowish to pale-yellowish transparent solution.

### Example 7

| | |
|---|---|
| Rebamipide | 1 g |
| Polyvinylpyrrolidone (K-25) | 1 g |
| Boric acid | 1.5 g |
| Meglumine | 5 g |
| Glycerin | 1.046 g |
| Hydrochloric acid | q.s. (to pH=8.2) |
| Purified water | q.s. |
| Total | 100 mL |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a colorless to light-yellowish transparent solution.

### Example 8

| | |
|---|---|
| Rebamipide | 1 g |
| Polyvinylpyrrolidone (K-25) | 6 g |
| Boric acid | 1.5 g |
| Meglumine | 4.7 g |
| Glycerin | 0.676 g |
| Hydrochloric acid | q.s. (to pH=8.0) |
| Purified water | q.s. |
| Total | 100 mL |

To a moderate amount of purified water were added rebamipide, polyvinylpyrrolidone (K-25), boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a light-yellowish to pale-yellowish transparent solution.

### Example 9

A yellowish to pale-yellowish transparent pharmaceutical composition was prepared in a similar manner to Example 1, using polyvinylpyrrolidone (K-17) in place of polyvinylpyrrolidone (K-25).

### Example 10

A light-yellowish to pale-yellowish transparent pharmaceutical composition was prepared in a similar manner to Example 2, using polyvinylpyrrolidone (K-17) in place of polyvinylpyrrolidone (K-25).

### Example 11

A colorless to light-yellowish transparent pharmaceutical composition was prepared in a similar manner to Example 3, using polyvinylpyrrolidone (K-17) in place of polyvinylpyrrolidone (K-25).

### Example 12

A light-yellowish to pale-yellowish transparent pharmaceutical composition was prepared in a similar manner to Example 4, using polyvinylpyrrolidone (K-17) in place of polyvinylpyrrolidone (K-25).

### Example 13

A light-yellowish to pale-yellowish transparent pharmaceutical composition was prepared in a similar manner to Example 5, using polyvinylpyrrolidone (K-17) in place of polyvinylpyrrolidone (K-25).

### Example 14

A light-yellowish to pale-yellowish transparent pharmaceutical composition was prepared in a similar manner to Example 6, using polyvinylpyrrolidone (K-17) in place of polyvinylpyrrolidone (K-25).

### Example 15

A colorless to light-yellowish transparent pharmaceutical composition was prepared in a similar manner to Example 7, using polyvinylpyrrolidone (K-17) in place of polyvinylpyrrolidone (K-25).

### Example 16

A light-yellowish to pale-yellowish transparent pharmaceutical composition was prepared in a similar manner to Example 8, using polyvinylpyrrolidone (K-17) in place of polyvinylpyrrolidone (K-25).

### Example 17

A light-yellowish to pale-yellowish transparent pharmaceutical composition was prepared in a similar manner to Example 1, using citric acid in place of hydrochloric acid.

### Example 18

A light-yellowish to pale-yellowish transparent pharmaceutical composition was prepared in a similar manner to Example 2, using citric acid in place of hydrochloric acid.

### Example 19

A colorless to light-yellowish transparent pharmaceutical composition was prepared in a similar manner to Example 3, using citric acid in place of hydrochloric acid.

### Example 20

A light-yellowish to pale-yellowish transparent pharmaceutical composition was prepared in a similar manner to Example 4, using citric acid in place of hydrochloric acid.

### Example 21

A light-yellowish to pale-yellowish transparent pharmaceutical composition was prepared in a similar manner to Example 5, using citric acid in place of hydrochloric acid.

### Example 22

A light-yellowish to pale-yellowish transparent pharmaceutical composition was prepared in a similar manner to Example 6, using citric acid in place of hydrochloric acid.

### Example 23

A colorless to light-yellowish transparent pharmaceutical composition was prepared in a similar manner to Example 7, using citric acid in place of hydrochloric acid.

### Example 24

A light-yellowish to pale-yellowish transparent pharmaceutical composition was prepared in a similar manner to Example 8, using citric acid in place of hydrochloric acid.

### Example 25

A light-yellowish to pale-yellowish transparent pharmaceutical composition was prepared in a similar manner to Example 1, using sucrose (2.889 g) in place of glycerin (0.722 g).

### Example 26

A light-yellowish to pale-yellowish transparent pharmaceutical composition was prepared in a similar manner to Example 2, using sucrose (4.741 g) in place of glycerin (1.185 g).

### Example 27

A colorless to light-yellowish transparent pharmaceutical composition was prepared in a similar manner to Example 3, using sucrose (3.185 g) in place of glycerin (0.796 g).

### Example 28

A light-yellowish to pale-yellowish transparent pharmaceutical composition was prepared in a similar manner to Example 4, using sucrose (1.593 g) in place of glycerin (0.398 g).

### Example 29

A light-yellowish to pale-yellowish transparent pharmaceutical composition was prepared in a similar manner to Example 5, using sucrose (3.963 g) in place of glycerin (0.991 g).

### Example 30

A light-yellowish to pale-yellowish transparent pharmaceutical composition was prepared in a similar manner to Example 6, using sucrose (5.556 g) in place of glycerin (1.389 g).

### Example 31

A colorless to light-yellowish transparent pharmaceutical composition was prepared in a similar manner to Example 7, using sucrose (4.185 g) in place of glycerin (1.046 g).

### Example 32

A light-yellowish to pale-yellowish transparent pharmaceutical composition was prepared in a similar manner to Example 8, using sucrose (2.704 g) in place of glycerin (0.676 g).

### Example 33

A light-yellowish to pale-yellowish transparent pharmaceutical composition was prepared in a similar manner to Example 1, using sucrose (2.889 g) in place of glycerin (0.722 g) and using citric acid in place of hydrochloric acid.

### Example 34

A light-yellowish to pale-yellowish transparent pharmaceutical composition was prepared in a similar manner to Example 5, using sucrose (3.963 g) in place of glycerin (0.991 g) and using citric acid in place of hydrochloric acid.

### Example 35

A light-yellowish to pale-yellowish transparent pharmaceutical composition was prepared in a similar manner to Example 1, using sucrose (2.889 g) in place of glycerin (0.722 g) and using phosphoric acid in place of hydrochloric acid.

### Example 36

A light-yellowish to pale-yellowish transparent pharmaceutical composition was prepared in a similar manner to Example 5, using sucrose (3.963 g) in place of glycerin (0.991 g) and using phosphoric acid in place of hydrochloric acid.

### Example 37

| | |
|---|---|
| Rebamipide | 2 g |
| Boric acid | 1.5 g |
| Meglumine | 6.2 g |
| Glycerin | 0.861 g |
| Hydrochloric acid | q.s. (to pH=8.7) |
| Purified water | q.s. |
| Total | 100 mL |

To a moderate amount of purified water were added rebamipide, boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a colorless transparent solution.

### Example 38

| | |
|---|---|
| Rebamipide | 2 g |
| Boric acid | 1 g |
| Meglumine | 4.5 g |
| Glycerin | 1.259 g |
| Hydrochloric acid | q.s. (to pH=8.7) |
| Purified water | q.s. |
| Total | 100 mL |

To a moderate amount of purified water were added rebamipide, boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a colorless transparent solution.

### Example 39

| | |
|---|---|
| Rebamipide | 1 g |
| Boric acid | 1.5 g |
| Meglumine | 5.2 g |
| Glycerin | 1.287 g |
| Hydrochloric acid | q.s. (to pH=8.4) |
| Purified water | q.s. |
| Total | 100 mL |

To a moderate amount of purified water were added rebamipide, boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a colorless transparent solution.

### Example 40

| | |
|---|---|
| Rebamipide | 1 g |
| Boric acid | 1 g |
| Meglumine | 3.6 g |
| Glycerin | 1.565 g |
| Hydrochloric acid | q.s. (to pH=8.0) |
| Purified water | q.s. |
| Total | 100 mL |

To a moderate amount of purified water were added rebamipide, boric acid, meglumine, and glycerin while stirring, and the pH of the resulting solution was adjusted with hydrochloric acid. The stirring was done with a low-speed propeller stirrer, without a high-speed stirrer such as a homomixer and a homogenizer. Every ingredient was easily dissolved with the low-speed propeller stirrer. Then, the resulting solution was aseptically filtered through a 0.2 µm filter to provide a pharmaceutical composition. The pharmaceutical composition was a colorless transparent solution.

### Example 41

A colorless transparent pharmaceutical composition was prepared in a similar manner to Example 37, using citric acid in place of hydrochloric acid.

### Example 42

A colorless transparent pharmaceutical composition was prepared in a similar manner to Example 39, using citric acid in place of hydrochloric acid.

### Example 43

A colorless transparent pharmaceutical composition was prepared in a similar manner to Example 37, using sucrose (3.444 g) in place of glycerin (0.861 g).

### Example 44

A colorless transparent pharmaceutical composition was prepared in a similar manner to Example 39, using sucrose (5.148 g) in place of glycerin (1.287 g).

### Example 45

A colorless transparent pharmaceutical composition was prepared in a similar manner to Example 37, using sucrose (3.444 g) in place of glycerin (0.861 g) and using citric acid in place of hydrochloric acid.

### Example 46

A colorless transparent pharmaceutical composition was prepared in a similar manner to Example 39, using sucrose (5.148 g) in place of glycerin (1.287 g) and using citric acid in place of hydrochloric acid.

### Example 47

A colorless transparent pharmaceutical composition was prepared in a similar manner to Example 37, using sucrose (3.444 g) in place of glycerin (0.861 g) and using phosphoric acid in place of hydrochloric acid.

### Example 48

A colorless transparent pharmaceutical composition was prepared in a similar manner to Example 39, using sucrose (5.148 g) in place of glycerin (1.287 g) and using phosphoric acid in place of hydrochloric acid.

The compositions of Examples 49 - 52 and Comparative examples 1 - 5 shown in the following Table 1 were prepared in the same manner as Example 1, using each ingredient and each amount thereof described in Table 1, each of which was a transparent pharmaceutical composition having a pH range of 7 - 9. The compositions were stored in a refrigerator for 4 weeks and then each aspect of the compositions was observed and recorded. In the result, a precipitate or gel was formed in the pharmaceutical compositions of Comparative examples 1 - 5 which contained an inorganic cation. While, in the pharmaceutical compositions of Examples 49 - 52 which did not contain any inorganic cation, a precipitate or gel was not formed at all.

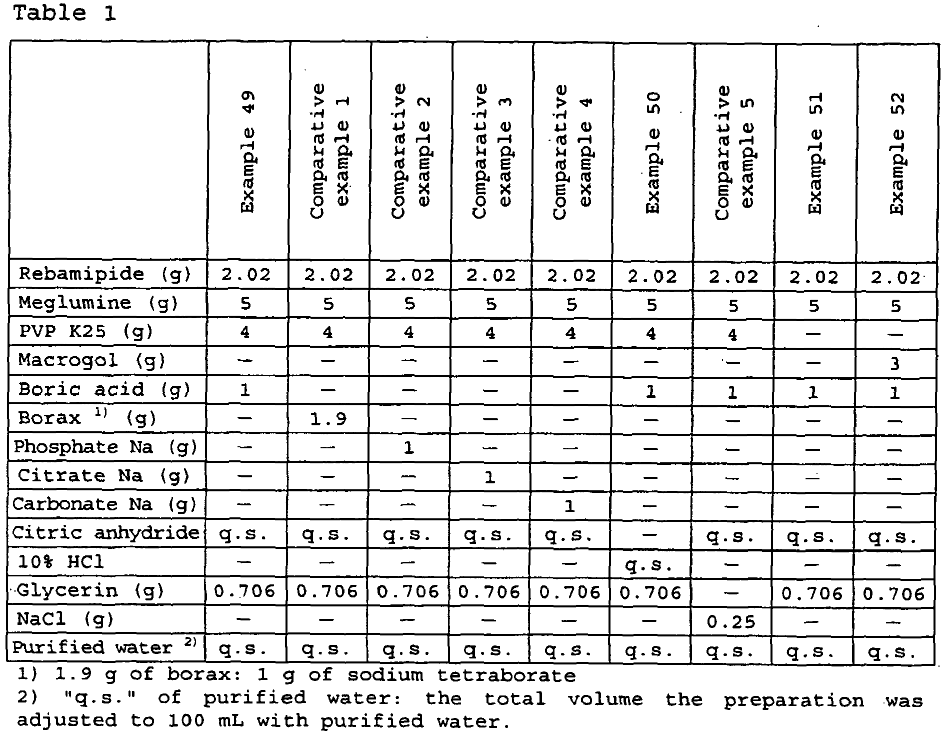

In order to confirm the good transparency and the good permeability of the present pharmaceutical composition, Example 1, Example 37, Comparative example 6 according to WO 2008/050896, and Comparative example 7 according to WO 2006/052018 were prepared. Shortly after each composition was prepared, each the aspect, the optical transparency at 640 nm, and the filtration performance over a 0.2 µm membrane filter were observed and recorded. After the compositions were stood at room temperature for four weeks, each the aspect was observed and recorded.

The results are shown in Table 2. In the present pharmaceutical compositions (Examples 1 and 37), the optical transparency thereof was not interrupted, the filtration performance thereof was good, and the aspect thereof was not changed even after 4 weeks. While, the pharmaceutical composition of Comparative example 6 was milky white, i.e. not transparent, and it was hard to be filtered. The pharmaceutical composition of Comparative example 7 was white, and it could not be filtered since the filter was blocked with its crystal. In addition, the pharmaceutical composition of Comparative example 7 which was stood for 4 weeks had to be vigorously shaken for redispersion since the crystal was deposited.

### Comparative example 6 (Preparation of suspension containing 2 W/V % crystalline rebamipide)

To 700 mL of an aqueous sodium hydroxide solution prepared by dissolving 4.4 g of sodium hydroxide in purified water was added 20 g of rebamipide. The mixture was dissolved by heating and then the solution was cooled to 30 - 40°C. On the other hand, 12 mL of 10 N hydrochloric acid, 68 mL of purified water and 200 mL of aqueous 10 W/V % hydroxypropylmethylcellulose (manufactured by Shin-Etsu Chemical Co.,Ltd, TC-5E) were mixed and the solution was cooled in an ice bath. To the solution stirred at 1400 rpm, the above sodium hydroxide solution containing rebamipide was gradually poured under sonication to deposit a crystal of rebamipide. After the crystal was deposited, the stirring speed was picked up to 3000 rpm, and the mixture was stirred for additional 20 minutes. After completing the deposition, the pH of the mixture was adjusted to 6 - 6.5 with 5 N sodium hydroxide, and the total volume thereof was adjusted to 1 liter with purified water to provide the desired aqueous suspension containing crystalline rebamipide.

### Comparative example 7 (Preparation of suspension containing 2 % (W/V) rebamipide)

To 80 mL of purified water were added 0.5 g of partially-saponified polyvinyl alcohol (manufactured by KURARAY CI.,LTD, Poval 224C), 0.11 g of citric acid, 0.146 g of sodium citrate, 0.715 g of sodium chloride and 0.180 g of potassium chloride, and the mixture was dissolved by heating. After cooling the solution, the solution was filtrated. To the filtrate was added rebamipide, and the resulting mixture was stirred. Then, the total volume thereof was adjusted to 100 mL with purified water to provide the desired aqueous suspension.

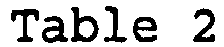

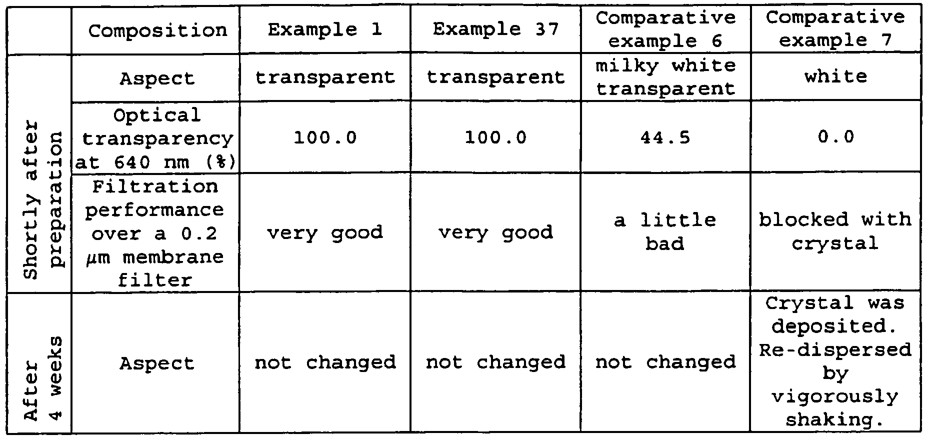

### Antimicrobial effectiveness test

### (Bacterial strains)

The following strains were used as an inoculum of the test.

| | |
|---|---|
| bacteria: | *Escherichia Coli* NBRC 3972 |
| | *Pseudomonas aeruginosa* NBRC 13275 |
| | *Staphylococcus aureus* NBRC 13276 |
| yeast and molds: | *Candida albicans* NBRC 1594 |
| | *Aspergillus niger* NBRC 9455 |

### (Test samples)

Examples 53 - 58 were prepared in a similar manner to Example 1 using ingredients listed in Table 3.

**Table 3. The compositions for antimicrobial effectiveness test**

| | Example 53 | Example 54 | Example 55 | Example 56 | Example 57 | Example 58 |
|---|---|---|---|---|---|---|
| Rebamipide | 2 g | 2 g | 2 g | 2 g | 2 g | 2 g |
| PVP (K25) | - | - | 3 g | 3 g | - | - |
| PVP (K17) | - | - | - | - | 3 g | 3 g |
| Boric acid | 1.5 g | 2 g | 1.5 g | 2 g | 1.5 g | 1.2 g |
| Meglumine | 6.2 g | 7.9 g | 5.9 g | 7.4 g | 5.7 g | 4.8 g |
| Glycerin | 0.972 g | 0.616 g | 0.844 g | 0.5 g | 0.819 g | 1.05 g |
| 10% HCl (pH) | q.s. (ca 8.7) | q.s. (ca 8.7) | q.s. (ca 8.3) | q.s. (ca 8.3) | q.s. (ca 8.3) | q.s. (ca 8.3) |
| Purified water | q.s. | q.s. | q.s. | q.s. | q.s. | q.s. |
| Total | 100 mL | 100 mL | 100 mL | 100 mL | 100 mL | 100 mL |

| | | | | | | |
|---|---|---|---|---|---|---|
| Note) PVP means polyvinylpyrrolidone. | | | | | | |

### (Method)

Each inoculum was aseptically added to Examples 53 - 58 at 10⁵ - 10⁶ cfu/mL, and each composition was uniformly mixed to provide each test sample. These samples were stored at 20 - 25°C protected from light. On 0, 14 and 28 days, each 1 mL was sampled from each test sample, and measured as to its viable cell count.

The measure of viable cell count of bacteria was carried out as follows. Each of the sample solutions was diluted in a tenfold dilutions with SCDLP medium. 1 mL of each grade of the dilutions was plated and 15 - 20 mL of SCDLP agar medium was added thereto. After cultivating the medium at 30 - 35 °C for 5 days, the measure of viable cell count was carried out. A plate wherein the viable cell count was not more than 300 was selected and the number of the count was recorded.

The measure of viable cell count of yeast and molds was carried out as follows. Each of the sample solutions was diluted in a tenfold dilutions with GPLP medium. 1 mL of each grade of the dilutions was plated and 15 - 20 mL of GPLP agar medium was added thereto. After cultivating the medium at 20 - 25 °C for 5 days, the measure of viable cell count was carried out. A plate wherein the viable cell count was not more than 100 was selected and the number of the count was recorded.

The viable cell count of each sample solution was provided by multiplying the recorded number by its dilution rate, and additionally the percentage of the viable cell count per the initial viable cell count was calculated.

### (Determination)

For bacteria, the determination of "acceptable" was given; in case that the percentage on 14th day was not more than 0.1 %; and the percentage on 28th day was the same level as the 14th day's result or less. The determination of the antimicrobial effectiveness was judged as "conforming" in case that the both were "acceptable".

For yeast and molds, the determination of "acceptable" was given, in case that the percentage was the same level as initial or less. The determination of the antimicrobial effectiveness was judged as "conforming" in case that the both results on 14th day and 28th day were "acceptable".

### (Result)

All Examples 53 - 58 were judged to have antimicrobial effectiveness.

**Table 4. The result of antimicrobial effectiveness test**

| | Bacterial strain | Results | | Antimicrobial effectiveness |
|---|---|---|---|---|
| | | After 14 days | After 28 days | |
| Example 53 | *E. Coli* | acceptable | acceptable | conforming |
| | *P*. *aeruginosa* | acceptable | acceptable | conforming |
| | *S*. *aureus* | acceptable | acceptable | conforming |
| | *C*. *albicans* | acceptable | acceptable | conforming |
| | *A. niger* | acceptable | acceptable | conforming |
| Example 54 | *E*. *Coli* | acceptable | acceptable | conforming |
| | *P. aeruginosa* | acceptable | acceptable | conforming |
| | *S*. *aureus* | acceptable | acceptable | conforming |
| | *C*. *albicans* | acceptable | acceptable | conforming |
| | *A. niger* | acceptable | acceptable | conforming |
| Example 55 | *E. Coli* | acceptable | acceptable | conforming |
| | *P*. *aeruginosa* | acceptable | acceptable | conforming |
| | *S*. aureus | acceptable | acceptable | conforming |
| | *C*. *albicans* | acceptable | acceptable | conforming |
| | *A. niger* | acceptable | acceptable | conforming |
| Example 56 | *E. Coli* | acceptable | acceptable | conforming |
| | *P*. *aeruginosa* | acceptable | acceptable | conforming |
| | *S*. *aureus* | acceptable | acceptable | conforming |
| | *C*. *albicans* | acceptable | acceptable | conforming |
| | *A*. *niger* | acceptable | acceptable | conforming |
| Example 57 | *E. Coli* | acceptable | acceptable | conforming |
| | *P*. *aeruginosa* | acceptable | acceptable | conforming |
| | *S*. *aureus* | acceptable | acceptable | conforming |
| | *C*. *albicans* | acceptable | acceptable | conforming |
| | *A. niger* | acceptable | acceptable | conforming |
| Example 58 | *E*. *Coli* | acceptable | acceptable | conforming |
| | *P*. *aeruginosa* | acceptable | acceptable | conforming |
| | *S*. *aureus* | acceptable | acceptable | conforming |
| | *C*. *albicans* | acceptable | acceptable | conforming |
| | *A. niger* | acceptable | acceptable | conforming |

## Claims

1. A pharmaceutical composition comprising (1) rebamipide, (2) an amino sugar, and (3) a buffer agent, which has no inorganic cation.

2. The pharmaceutical composition of claim 1 wherein the amino sugar is at least one selected from the group consisting of D-glucosamine and meglumine.

3. The pharmaceutical composition of claim 1 or 2 wherein the buffer agent is at least one selected from the group consisting of boric acid, phosphoric acid and an amino acid.

4. The pharmaceutical composition of any one of claims 1 to 3, further comprising a pH adjuster.

5. The pharmaceutical composition of any one of claims 1 to 4, further comprising a solubilizing agent.

6. The pharmaceutical composition of claim 5 wherein the solubilizing agent is at least one selected from the group consisting of polyvinylpyrrolidone and macrogol.

7. The pharmaceutical composition of any one of claims 1 to 6 wherein the pharmaceutical composition is an ophthalmic pharmaceutical composition.

8. The pharmaceutical composition of any one of claims 1 to 7 for use in the treatment of dry eye by topical administration to the eye.

## Patentansprüche

1. Pharmazeutische Zusammensetzung, die (1) Rebamipid, (2) einen Aminozucker und (3) ein Puffermittel, das kein anorganisches Kation aufweist, umfasst.

2. Pharmazeutische Zusammensetzung gemäß Anspruch 1, wobei der Aminozucker wenigstens einer ist, der aus der Gruppe ausgewählt ist, die aus D-Glucosamin und Meglumin besteht.

3. Pharmazeutische Zusammensetzung gemäß Anspruch 1 oder 2, wobei das Puffermittel wenigstens eines ist, das aus der Gruppe ausgewählt ist, die aus Borsäure, Phosphorsäure und einer Aminosäure besteht.

4. Pharmazeutische Zusammensetzung gemäß einem der Ansprüche 1 bis 3, die weiterhin einen pH-Regulator umfasst.

5. Pharmazeutische Zusammensetzung gemäß einem der Ansprüche 1 bis 4, die weiterhin einen Lösungsvermittler umfasst.

6. Pharmazeutische Zusammensetzung gemäß Anspruch 5, wobei der Lösungsvermittler wenigstens einer ist, der aus der Gruppe ausgewählt ist, die aus Polyvinylpyrrolidon und Macrogol besteht.

7. Pharmazeutische Zusammensetzung gemäß einem der Ansprüche 1 bis 6, wobei die pharmazeutische Zusammensetzung eine ophthalmische pharmazeutische Zusammensetzung ist.

8. Pharmazeutische Zusammensetzung gemäß einem der Ansprüche 1 bis 7 zur Verwendung bei der Behandlung von Xerophthalmie durch topische Verabreichung an das Auge.

## Revendications

1. Composition pharmaceutique comprenant (1) du rébamipide, (2) un amino sucre et (3) un agent tampon, qui n'a pas de cation inorganique.

2. Composition pharmaceutique selon la revendication 1 où l'amino sucre est au moins un choisi dans le groupe consistant en la D-glucosamine et la méglumine.

3. Composition pharmaceutique selon la revendication 1 ou 2 où l'agent tampon est au moins un choisi dans le groupe consistant en l'acide borique, l'acide phosphorique et un aminoacide.

4. Composition pharmaceutique selon l'une quelconque des revendications 1 à 3, comprenant en outre un agent d'ajustement du pH.

5. Composition pharmaceutique selon l'une quelconque des revendications 1 à 4, comprenant en outre un agent solubilisant.

6. Composition pharmaceutique selon la revendication 5 où l'agent solubilisant est au moins un choisi dans le groupe consistant en la polyvinylpyrrolidone et le macrogol.

7. Composition pharmaceutique selon l'une quelconque des revendications 1 à 6 où la composition pharmaceutique est une composition pharmaceutique ophtalmique.

8. Composition pharmaceutique selon l'une quelconque des revendications 1 à 7 destinée à être utilisée dans le traitement de l'oeil sec par administration topique à l'oeil.
